# EUROPEAN PATENT APPLICATION

(11) **EP 1 541 135 A1**
(43) Date of publication of application: **15.06.2005**
(21) Application number: 03761834.5
(22) Date of filing: 27.06.2003
(51) Int. Cl.: A61K 9/51, A61K 47/42, A61K 48/00, A61P 1/16, A61P 35/00, A61P 43/00

(54) **HOLLOW NANOPARTICLE HAVING MODIFIED CYSTEINE RESIDUE AND DRUG WITH THE USE THEREOF**

(30) Priority: 28.06.2002 JP 2002191386; 27.06.2003 JP 2003183863
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP); TADA, Hiroko, Okayama-shi, Okayama 700-0016 (JP)
(74) Representative: Bentz, Jean-Paul
(86) International application number: PCT/JP2003/008244
(87) International publication number: WO 2004/002459

(57) **Abstract**

The invention provides hollow nanoparticles of a protein with the ability to recognize specific cells such as the hepatocytes and to form particles (for example, hepatitis B virus surface-antigen protein), wherein the protein has a cysteine residue substituted to a different amino acid. The hollow nanoparticles have a stable particle structure and can be used to efficiently transfer substances to specific target cells or tissues. The invention also provides a drug using the hollow nanoparticles.

SEQUENCE LISTING

<110> Japan Science and Technology Corporation

<120> Hollow nano-particles composed of cysteine-modified proteins, and
their use as a therapentic drug

<130>P023P05

<150> JP2002-191386
<151> 2002-6-28

<150> JP2003-183863
<151> 2003-6-27

<160> 36

<170> PatentIn Ver. 2.1

<210> 1
<211> 1218
<212> DNA
<213> Hepatitis B virus

<220>
<221> CDS
<222> (1)..(1218)

<400> 1
atg aga tct ttg ttg atc ttg gtt ttg tgt ttc ttg cca ttg gct gct 48
Met Arg Ser Leu Leu Ile Leu Val Leu Cys Phe Leu Pro Leu Ala Ala
1               5                   10                  15

ttg ggt aag gtt cga caa ggc atg ggg acg aat ctt tct gtt ccc aat 96
Leu Gly Lys Val Arg Gln Gly Met Gly Thr Asn Leu Ser Val Pro Asn

            20                  25                  30

cct ctg gga ttc ttt ccc gat cac cag ttg gac cct gcg ttc gga gcc 144
Pro Leu Gly Phe Phe Pro Asp His Gln Leu Asp Pro Ala Phe Gly Ala
        35                  40                  45

aac tca aac aat cca gat tgg gac ttc aac ccc aac aag gat caa tgg 192
Asn Ser Asn Asn Pro Asp Trp Asp Phe Asn Pro Asn Lys Asp Gln Trp
    50                  55                  60

cca gag gca aat cag gta gga gcg gga gca ttc ggg cca ggg ttc acc 240
Pro Glu Ala Asn Gln Val Gly Ala Gly Ala Phe Gly Pro Gly Phe Thr
65                  70                  75                  80

cca cca cac ggc ggt ctt ttg ggg tgg agc cct cag gct cag ggc ata 288
Pro Pro His Gly Gly Leu Leu Gly Trp Ser Pro Gln Ala Gln Gly Ile
                85                  90                  95

ttg aca aca gtg cca gca gca cct cct cct gcc tcc acc aat cgg cag 336
Leu Thr Thr Val Pro Ala Ala Pro Pro Pro Ala Ser Thr Asn Arg Gln
            100                 105                 110

tca gga aga cag cct act ccc atc tct cca cct cta aga gac agt cat 384
Ser Gly Arg Gln Pro Thr Pro Ile Ser Pro Pro Leu Arg Asp Ser His
        115                 120                 125

cct cag gcc atg cag tgg aat tcc aca aca ttc cac caa gct ctg cta 432
Pro Gln Ala Met Gln Trp Asn Ser Thr Thr Phe His Gln Ala Leu Leu
    130                 135                 140

gat ccc aga gtg agg ggc cta tat ttt cct gct ggt ggc tcc agt tcc 480
Asp Pro Arg Val Arg Gly Leu Tyr Phe Pro Ala Gly Gly Ser Ser Ser
145                 150                 155                 160

gga aca gta aac cct gtt ccg act act gcc tca ccc ata tct ggg gac 528
Gly Thr Val Asn Pro Val Pro Thr Thr Ala Ser Pro Ile Ser Gly Asp

                165                 170                 175

cct gca ccg aac atg gag aac aca aca tca gga ttc cta gga ccc ctg 576
Pro Ala Pro Asn Met Glu Asn Thr Thr Ser Gly Phe Leu Gly Pro Leu
            180                 185                 190

ctc gtg tta cag gcg ggg ttt ttc ttg ttg aca aga atc ctc aca ata 624
Leu Val Leu Gln Ala Gly Phe Phe Leu Leu Thr Arg Ile Leu Thr Ile
        195                 200                 205

cca cag agt cta gac tcg tgg tgg act tct ctc aat ttt cta ggg gga 672
Pro Gln Ser Leu Asp Ser Trp Trp Thr Ser Leu Asn Phe Leu Gly Gly
    210                 215                 220

gca ccc acg tgt cct ggc caa aat tcg cag tcc cca acc tcc aat cac 720
Ala Pro Thr Cys Pro Gly Gln Asn Ser Gln Ser Pro Thr Ser Asn His
225                 230                 235                 240

tca cca acc tct tgt cct cca att tgt cct ggc tat cgc tgg atg tgt 768
Ser Pro Thr Ser Cys Pro Pro Ile Cys Pro Gly Tyr Arg Trp Met Cys
                245                 250                 255

ctg cgg cgt ttt atc ata ttc ctc ttc atc ctg ctg cta tgc ctc atc 816
Leu Arg Arg Phe Ile Ile Phe Leu Phe Ile Leu Leu Leu Cys Leu Ile
            260                 265                 270

ttc ttg ttg gtt ctt ctg gac tac caa ggt atg ttg ccc gtt tgt cct 864
Phe Leu Leu Val Leu Leu Asp Tyr Gln Gly Met Leu Pro Val Cys Pro
        275                 280                 285

cta ctt cca gga aca tca acc acc agc acg ggg cca tgc aag acc tgc 912
Leu Leu Pro Gly Thr Ser Thr Thr Ser Thr Gly Pro Cys Lys Thr Cys
    290                 295                 300

acg att cct gct caa gga acc tct atg ttt ccc tct tgt tgc tgt aca 960
Thr Ile Pro Ala Gln Gly Thr Ser Met Phe Pro Ser Cys Cys Cys Thr

305                 310                 315                 320

aaa cct tcg gac gga aac tgc act tgt att ccc atc cca tca tcc tgg 1008
Lys Pro Ser Asp Gly Asn Cys Thr Cys Ile Pro Ile Pro Ser Ser Trp
                325                 330                 335

gct ttc gca aga ttc cta tgg gag tgg gcc tca gtc cgt ttc tcc tgg 1056
Ala Phe Ala Arg Phe Leu Trp Glu Trp Ala Ser Val Arg Phe Ser Trp
            340                 345                 350

ctc agt tta cta gtg cca ttt gtt cag tgg ttc gta ggg ctt tcc ccc 1104
Leu Ser Leu Leu Val Pro Phe Val Gln Trp Phe Val Gly Leu Ser Pro
        355                 360                 365

act gtt tgg ctt tca gtt ata tgg atg atg tgg tat tgg ggg cca agt 1152
Thr Val Trp Leu Ser Val Ile Trp Met Met Trp Tyr Trp Gly Pro Ser
    370                 375                 380

ctg tac aac atc ttg agt ccc ttt tta cct cta tta cca att ttc ttt 1200
Leu Tyr Asn Ile Leu Ser Pro Phe Leu Pro Leu Leu Pro Ile Phe Phe
385                 390                 395                 400

tgt ctt tgg gta tat att 1218
Cys Leu Trp Val Tyr Ile
                405

<210> 2
<211> 406
<212> PRT
<213> Hepatitis B virus

<400> 2
Met Arg Ser Leu Leu Ile Leu Val Leu Cys Phe Leu Pro Leu Ala Ala
1               5                   10                  15

Leu Gly Lys Val Arg Gln Gly Met Gly Thr Asn Leu Ser Val Pro Asn
            20                  25                  30

Pro Leu Gly Phe Phe Pro Asp His Gln Leu Asp Pro Ala Phe Gly Ala
        35                  40                  45

Asn Ser Asn Asn Pro Asp Trp Asp Phe Asn Pro Asn Lys Asp Gln Trp
    50                  55                  60

Pro Glu Ala Asn Gln Val Gly Ala Gly Ala Phe Gly Pro Gly Phe Thr
65                  70                  75                  80

Pro Pro His Gly Gly Leu Leu Gly Trp Ser Pro Gln Ala Gln Gly Ile
                85                  90                  95

Leu Thr Thr Val Pro Ala Ala Pro Pro Pro Ala Ser Thr Asn Arg Gln
            100                 105                 110

Ser Gly Arg Gln Pro Thr Pro Ile Ser Pro Pro Leu Arg Asp Ser His
        115                 120                 125

Pro Gln Ala Met Gln Trp Asn Ser Thr Thr Phe His Gln Ala Leu Leu
    130                 135                 140

Asp Pro Arg Val Arg Gly Leu Tyr Phe Pro Ala Gly Gly Ser Ser Ser
145                 150                 155                 160

Gly Thr Val Asn Pro Val Pro Thr Thr Ala Ser Pro Ile Ser Gly Asp
                165                 170                 175

Pro Ala Pro Asn Met Glu Asn Thr Thr Ser Gly Phe Leu Gly Pro Leu
            180                 185                 190

Leu Val Leu Gln Ala Gly Phe Phe Leu Leu Thr Arg Ile Leu Thr Ile
        195                 200                 205

Pro Gln Ser Leu Asp Ser Trp Trp Thr Ser Leu Asn Phe Leu Gly Gly
    210                 215                 220

Ala Pro Thr Cys Pro Gly Gln Asn Ser Gln Ser Pro Thr Ser Asn His
225                 230                 235                 240

Ser Pro Thr Ser Cys Pro Pro Ile Cys Pro Gly Tyr Arg Trp Met Cys
                245                 250                 255

Leu Arg Arg Phe Ile Ile Phe Leu Phe Ile Leu Leu Leu Cys Leu Ile
            260                 265                 270

Phe Leu Leu Val Leu Leu Asp Tyr Gln Gly Met Leu Pro Val Cys Pro
        275                 280                 285

Leu Leu Pro Gly Thr Ser Thr Thr Ser Thr Gly Pro Cys Lys Thr Cys
    290                 295                 300

Thr Ile Pro Ala Gln Gly Thr Ser Met Phe Pro Ser Cys Cys Cys Thr
305                 310                 315                 320

Lys Pro Ser Asp Gly Asn Cys Thr Cys Ile Pro Ile Pro Ser Ser Trp
                325                 330                 335

Ala Phe Ala Arg Phe Leu Trp Glu Trp Ala Ser Val Arg Phe Ser Trp
            340                 345                 350

Leu Ser Leu Leu Val Pro Phe Val Gln Trp Phe Val Gly Leu Ser Pro
        355                 360                 365

Thr Val Trp Leu Ser Val Ile Trp Met Met Trp Tyr Trp Gly Pro Ser
    370                 375                 380

Leu Tyr Asn Ile Leu Ser Pro Phe Leu Pro Leu Leu Pro Ile Phe Phe
385                 390                 395                 400

Cys Leu Trp Val Tyr Ile
                405

<210> 3
<211> 26
<212> DNA
<213> Artificial Sequence

<220>
<223> Description of Artificial Sequence:Artificially
Synthesized Primer Sequence

<400> 3
gcacccacgt ctcctggcca aaattc 26

<210> 4
<211> 26
<212> DNA
<213> Artificial Sequence

<220>
<223> Description of Artificial Sequence:Artificially
Synthesized Primer Sequence

<400> 4
gaattttggc caggagacgt gggtgc 26

<210> 5
<211> 26
<212> DNA
<213> Artificial Sequence

<220>
<223> Description of Artificial Sequence:Artificially
      Synthesized Primer Sequence

<400> 5
tcaccaacct ctagtcctcc aatttg                                          26


<210> 6
<211> 26
<212> DNA
<213> Artificial Sequence

<220>
<223> Description of Artificial Sequence:Artificially
      Synthesized Primer Sequence

<400> 6
caaattggag gactagaggt tggtga                                          26


<210> 7
<211> 28
<212> DNA
<213> Artificial Sequence

<220>
<223> Description of Artificial Sequence:Artificially
      Synthesized Primer Sequence

<400> 7
cttgtcctcc aataagtcct ggctatcg                                        28


<210> 8
<211> 28

<212> DNA
<213> Artificial Sequence

<220>
<223> Description of Artificial Sequence:Artificially
Synthesized Primer Sequence

<400> 8
cgatagccag gacttattgg aggacaag 28

<210> 9
<211> 30
<212> DNA
<213> Artificial Sequence

<220>
<223> Description of Artificial Sequence:Artificially
Synthesized Primer Sequence

<400> 9
tatcgctgga tggcgctgcg gcgttttatc 30

<210> 10
<211> 30
<212> DNA
<213> Artificial Sequence

<220>
<223> Description of Artificial Sequence:Artificially
Synthesized Primer Sequence

<400> 10
aaaacgccgc agcgccatcc agcgatagcc 30

<210> 11
<211> 28
<212> DNA
<213> Artificial Sequence

<220>
<223> Description of Artificial Sequence:Artificially
      Synthesized Primer Sequence

<400> 11
catcctgctg ctacccctca tcttcttg                                        28


<210> 12
<211> 28
<212> DNA
<213> Artificial Sequence

<220>
<223> Description of Artificial Sequence:Artificially
      Synthesized Primer Sequence

<400> 12
caagaagatg agggctagca gcaggatg                                        28


<210> 13
<211> 27
<212> DNA
<213> Artificial Sequence

<220>
<223> Description of Artificial Sequence:Artificially
      Synthesized Primer Sequence

<400> 13
atgttgcccg ttgcgcctct acttcca                                    27

<210> 14
<211> 27
<212> DNA
<213> Artificial Sequence

<220>
<223> Description of Artificial Sequence:Artificially
      Synthesized Primer Sequence

<400> 14
tggaagtaga ggcgcaacgg gcaacat                                    27

<210> 15
<211> 30
<212> DNA
<213> Artificial Sequence

<220>
<223> Description of Artificial Sequence:Artificially
      Synthesized Primer Sequence

<400> 15
agcacggggc cttcgaagac ctgcacgatt                                 30

<210> 16
<211> 30
<212> DNA
<213> Artificial Sequence

<220>

<223> Description of Artificial Sequence:Artificially
Synthesized Primer Sequence

<400> 16
gtgcaggtct tcgaaggccc cgtgctggtg 30

<210> 17
<211> 27
<212> DNA
<213> Artificial Sequence

<220>
<223> Description of Artificial Sequence:Artificially
Synthesized Primer Sequence

<400> 17
ccatgcaaga cctcgacgat tcctgct 27

<210> 18
<211> 27
<212> DNA
<213> Artificial Sequence

<220>
<223> Description of Artificial Sequence:Artificially
Synthesized Primer Sequence

<400> 18
agcaggaatc gtcgaggtct tgcatgg 27

<210> 19
<211> 25
<212> DNA

<213> Artificial Sequence

<220>
<223> Description of Artificial Sequence:Artificially
      Synthesized Primer Sequence

<400> 19
atgtttccct ctagttgctg tacaa                                          25


<210> 20
<211> 25
<212> DNA
<213> Artificial Sequence

<220>
<223> Description of Artificial Sequence:Artificially
      Synthesized Primer Sequence

<400> 20
ttgtacagca actagaggga aacat                                          25


<210> 21
<211> 25
<212> DNA
<213> Artificial Sequence

<220>
<223> Description of Artificial Sequence:Artificially
      Synthesized Primer Sequence

<400> 21
tttccctctt gcagctgtac aaaac                                          25

<210> 22
<211> 25
<212> DNA
<213> Artificial Sequence

<220>
<223> Description of Artificial Sequence:Artificially
Synthesized Primer Sequence

<400> 22
ttttgtacag ctgcaagagg gaaac 25

<210> 23
<211> 26
<212> DNA
<213> Artificial Sequence

<220>
<223> Description of Artificial Sequence:Artificially
Synthesized Primer Sequence

<400> 23
cctcttgttg ctcgacaaaa ccttcg 26

<210> 24
<211> 26
<212> DNA
<213> Artificial Sequence

<220>
<223> Description of Artificial Sequence:Artificially
Synthesized Primer Sequence

<400> 24

cgaaggtttt gtcgagcaac aagagg                                                26

<210> 25
<211> 26
<212> DNA
<213> Artificial Sequence

<220>
<223> Description of Artificial Sequence:Artificially
      Synthesized Primer Sequence

<400> 25
tcggacggaa acagcacttg tattcc                                                26

<210> 26
<211> 26
<212> DNA
<213> Artificial Sequence

<220>
<223> Description of Artificial Sequence:Artificially
      Synthesized Primer Sequence

<400> 26
ggaatacaag tgctgtttcc gtccga                                                26

<210> 27
<211> 28
<212> DNA
<213> Artificial Sequence

<220>
<223> Description of Artificial Sequence:Artificially

Synthesized Primer Sequence

<400> 27
cggaaactgc acggccattc ccatccca 28

<210> 28
<211> 28
<212> DNA
<213> Artificial Sequence

<220>
<223> Description of Artificial Sequence:Artificially
Synthesized Primer Sequence

<400> 28
tgggatggga atggccgtgc agtttccg 28

<210> 29
<211> 28
<212> DNA
<213> Artificial Sequence

<220>
<223> Description of Artificial Sequence:Artificially
Synthesized Primer Sequence

<400> 29
accaattttc tttgcgcttt gggtatac 28

<210> 30
<211> 28
<212> DNA
<213> Artificial Sequence

<220>
<223> Description of Artificial Sequence:Artificially
Synthesized Primer Sequence

<400> 30
gtatacccaa agcgcaaaga aaattggt 28

<210> 31
<211> 30
<212> DNA
<213> Artificial Sequence

<220>
<223> Description of Artificial Sequence:Artificially
Synthesized Primer Sequence

<400> 31
ccttcggacg gaaacagcac ggccattccc 30

<210> 32
<211> 30
<212> DNA
<213> Artificial Sequence

<220>
<223> Description of Artificial Sequence:Artificially
Synthesized Primer Sequence

<400> 32
gggaatggcc gtgctgtttc cgtccgaagg 30

<210> 33

<211> 25
<212> DNA
<213> Artificial Sequence

<220>
<223> Description of Artificial Sequence:Artificially
Synthesized Primer Sequence

<400> 33
tttccctctt gtagctcgac aaaac 25

<210> 34
<211> 25
<212> DNA
<213> Artificial Sequence

<220>
<223> Description of Artificial Sequence:Artificially
Synthesized Primer Sequence

<400> 34
gttttgtcga gctacaagag ggaaa 25

<210> 35
<211> 25
<212> DNA
<213> Artificial Sequence

<220>
<223> Description of Artificial Sequence:Artificially
Synthesized Primer Sequence

<400> 35
atgtttccct cttctagctc gacaa 25

<210> 36
<211> 25
<212> DNA
<213> Artificial Sequence

<220>
<223> Description of Artificial Sequence:Artificially
      Synthesized Primer Sequence

<400> 36
ttgtcgagct agaagaggga aacat                                          25

## Description

### TECHNICAL FIELD

The present invention relates to hollow nanoparticles for encapsulating a substance to be transferred into a cell for treating a disease. Specifically, the invention relates to a drug that allows the disease-treating substance encapsulated in particles to be specifically transferred into a specific cell or tissue.

### BACKGROUND ART

In the field of medicine, there has been active research on drugs that directly and effectively act on the affected area without causing serious side effects. One area of active research is a method known as a drug delivery system (DDS), in which active ingredients of drugs or other substances are specifically delivered to a target cell or tissue, where they can exhibit their effects.

A gene transfer method is one known example of a method of transferring a protein drug to a target cell or tissue. In the gene transfer method, an expression vector that has incorporated a gene for encoding the protein is transferred into a target cell by an electroporation method or other techniques. Inside the cell, the gene is expressed into the protein drug. However, the conventional gene transfer methods are not sufficient to specifically transfer the protein drug to a target cell or tissue.

Under these circumstances, the inventors of the present invention have previously proposed a method of specifically and safely delivering and transferring various substances (including genes, proteins, compounds) into a target cell or tissue, using hollow nanoparticles of a protein that has the ability to form particles and has incorporated a bio-recognizing molecule, as disclosed in International Publication with International Publication No. WO01/64930 (published on September 7, 2001) (hereinafter referred to as "International Publication WO01/64930"). However, the publication does not fully discuss how the method can be used to efficiently transfer these substances to a target cell or tissue.

The present invention was made in view of the foregoing problems, and an object of the invention is to provide hollow protein nanoparticles for specifically and efficiently transferring a substance into a target cell or tissue. The present invention also provides a drug containing hollow protein nanoparticles encapsulating a substance to be transferred into a cell.

### DISCLOSURE OF INVENTION

The inventors of the present invention accomplished the present invention by finding that a modified cysteine residue in the particle protein greatly improves uptake of a particle substance by a specific cell.

That is, the present invention discloses hollow nanoparticles of a particle-forming protein with the ability to recognize specific cells (for example, hepatocytes), wherein the protein has a modified cysteine residue.

An example of such a "particle-forming protein" is a hepatitis B virus surface-antigen protein. In eukaryotic cells, the protein is expressed as a membrane protein on the endoplasmic reticulum and accumulates thereon before it is released as particles. Hollow nanoparticles of the present invention may be produced by transforming a eukaryotic cell (for example, animal cells including mammal cells, or yeast cells) with a vector including a gene coding for a particle-forming protein, and then expressing the gene in the eukaryotic cell.

When the hepatitis B virus surface-antigen protein is used to form particles, the particles recognize the hepatocytes and deliver a substance contained in the particles specifically to the hepatocytes. Thus, with particles containing a substance (gene, etc.) for treating a liver disease, an effective therapeutic drug can be provided that effectively and specifically acts on the hepatocytes.

For example, the hepatitis B virus surface-antigen protein may be modified to lack its infectivity to the hepatocytes and display a growth factor or an antibody. Particles of such a modified protein can deliver a substance contained in the particles to specific cells other than the hepatocytes. For example, by displaying an antibody that specifically recognizes a certain cancer cell, the particles can recognize the cancer cell and deliver a substance contained in the particles specifically to the cancer cell.

The hepatitis B virus surface-antigen protein contains S protein (described later) that includes a total of 14 cysteine (Cys) residues associated with the oxidation-reduction of the protein. The Cys residues are believed to control the particle structure they form. However, the cysteine residues have a problem of instability. During the purification and/or preservation of the particles, the cysteine residues form excess disulfide bonds, causing the protein to polymerize by the randomly formed intermolecular and intramolecular disulfide crosslinkage. Such polymerization can be prevented by modifying some of the Cys residues that do not play important role in the control of the protein structure. (Alternatively, polymerization can be prevented by modifying other Cys residues that have essentially no influence on the ability to form particles or recognize cells.) This imparts stability to the particles and thereby improves the efficiency by which substances are encapsulated in the hollow nanoparticles. As a result, the substances can be efficiently transferred into a cell.

Specifically, it is preferable that substitution takes place at Cys residues 76, 90, 139, 147, 149, 221 and at least one of Cys residues 137 and 138 from the N-terminus of the amino acid sequence of the S protein in the hepatitis B virus surface-antigen protein.

It is preferable that the Cys residues be modified by substitution by other amino acids. Alternatively, the Cys residues may be modified by deletion. Being a transmembrane protein, the hepatitis B virus surface-antigen protein exists both inside and outside of the particles, across the lipid bilayer membrane. Among the Cys residues in the protein, it is preferable that those believed to exist inside the lipid bilayer membrane be replaced with hydrophobic amino acids (for example, alanine (Ala)), and that those believed to exist inside and outside of the particles be replaced with hydrophilic amino acids (for example, serine (Ser)).

The Cys residues can be modified in this manner to prepare modified hollow nanoparticles. For example, a gene encoding a particle-forming protein may be mutated and incorporated into a vector to transform a eukaryotic cell in which the mutated gene is expressed.

The present invention discloses a drug that contains a therapeutic substance in its hallow nanoparticles. The drug can be used by a convenient method of intravenous injection to effectively treat specific diseased cells or tissues. The drug is a great leap forward from conventional disease treatment methods in that it does not require large dose or any surgical operation in disease treatment including gene therapy, and that the risk of side effect is greatly reduced. The drug is therefore usable in clinical applications in its present form.

The present invention also discloses a treatment method for treating diseases through administration of the drug disclosed in the present invention.

### BRIEF DESCRIPTION OF DRAWINGS

Fig. 1 is a schematic diagram showing a HBsAg L protein forming hollow nanoparticles according to the present invention, in which Cys residues have not been replaced.
Fig. 2 represents an amino acid sequence of a HBsAg S protein forming hollow nanoparticles according to the present invention, in which Cys residues have not been replaced.
Fig. 3 illustrates a preparation method of a gene plasmid that encodes the HBsAg L protein forming hollow nanoparticles according to the present invention.
Fig. 4(a) and Fig. 4(b) are schematic diagrams showing lines representing the primary structure of a HBsAg protein forming hollow nanoparticles according to the present invention, in which Fig. 4(a) represents a HBsAg protein with the substitution of one Cys residue, and Fig. 4(b) represents a HBsAg protein with the substitution of two or more Cys residues.
Fig. 5 represents antigenicity of hollow nanoparticles according to the present invention, wherein the antigenicity has been calculated as relative values with respect to that of a wild-type held at 100.
Fig. 6(a) through Fig. 6(h) are electrophoretographs, showing the results of Western blotting on HBsAg particles according to the present invention under reduced conditions (Fig. 6(a) through Fig. 6(d)) and non-reduced conditions (Fig. 6(e) through Fig. 6(h)).
Fig. 7(a) and Fig. 7(b) represent gene transfer efficiency of a gene transferred into HepG2 cells using HBsAg particles according to the present invention.
Fig. 8(a) through Fig. 8(f) represent gene transfer efficiency of a gene transferred into HepG2 cells using BNP-Lm8, which is one form of HBsAg particles according to the present invention.
Fig. 9(a) through Fig. 9(c) represent gene transfer efficiency of a gene transferred into HepG2 cells using BNP-Lm8, which is one form of HBsAg particles according to the present invention, respectively magnifying Fig. 8(a), Fig. 8(e), and Fig. 8(f).
Fig. 10(a) through Fig. 10(d) represent gene transfer efficiency of a gene transferred into HepG2 cells using BNP-Lm8, which is one form of HBsAg particles according to the present invention, after the BNP-Lm8 was preserved for a week at 4°C.
Fig. 11(a) through Fig. 11(d) represent gene transfer efficiency of a gene transferred into HepG2 cells using BNP-Lm8, which is one form of HBsAg particles according to the present invention.
Fig. 12(a) through Fig. 12(d) represent gene transfer efficiency of a gene transferred into WiDr cells using BNP-Lm8, which is one form of HBsAg particles according to the present invention.
Fig. 13(a) through Fig. 13(h) compare gene transfer efficiencies of a gene transferred into HepG2 cells using BNP-Lm8 and BNP-Lm7b, which are different forms of HBsAg particles according to the present invention.

### BEST MODE FOR CARRYING OUT THE INVENTION

Hollow nanoparticles of the present invention contain modified cysteine residues in the protein forming the particles. The particle-forming protein may incorporate a bio-recognizing molecule (i.e., a molecule recognizing a specific cell) that enables a substance to be specifically delivered to a target cell or tissue. Examples of such particle-forming proteins include sub viral particles obtained from various viruses. A specific example is a hepatitis B virus (HBV) surface-antigen protein. (In the following, the hepatitis B virus surface-antigen protein may be denoted by "HBsAg.")

The protein particles of such a particle-forming protein may be obtained through the protein expression in eukaryotic cells. Specifically, in the eukaryotic cell, the particle-forming protein is expressed on the endoplasmic reticulum as a membrane protein and accumulates thereon before it is released as particles. The eukaryotic cell may be obtained from yeasts, or animals including mammals.

As will be described later in Examples, the inventors of the present invention have reported that the expression of HBV surface-antigen L protein in recombinant yeast cells produces ellipsoidal hollow particles with a minor axis of 20 nm and a major axis of 150 nm, with a large number of L proteins embedded in the yeast-derived lipid bilayer membrane (J. Biol. Chem., Vol. 267, No. 3, 1953-1961, 1992). The particles contain no HBV genome and lack the viral function. Therefore, the particles are very safe to the human body. Further, because the particles on the particle surface display hepatocyte-specific receptors that make the HBV highly infectious to the hepatocytes, the particles are highly effective as a carrier for delivering substances specifically to the hepatocytes.

The method of producing protein particles using recombinant yeasts is therefore suitable in efficiently producing the particles from a soluble protein in the yeast.

The HBsAg contains S protein (described later) includes a total of 14 cysteine (Cys) residues associated with the oxidation-reduction of the protein. The Cys residues are believed to control the particle structure they form. However, the cysteine residues have a problem of instability. During the purification and/or preservation of the particles, the cysteine residues form excess disulfide bonds, causing the protein to polymerize. The particles can be stabilized to improve their preservation property by modifying some of the Cys residues that do not play important role in the control of the protein structure. Further, with the Cys residues modified in this manner, substances can be more easily encapsulated in the hollow nanoparticles and thereby more efficiently transferred into the cell.

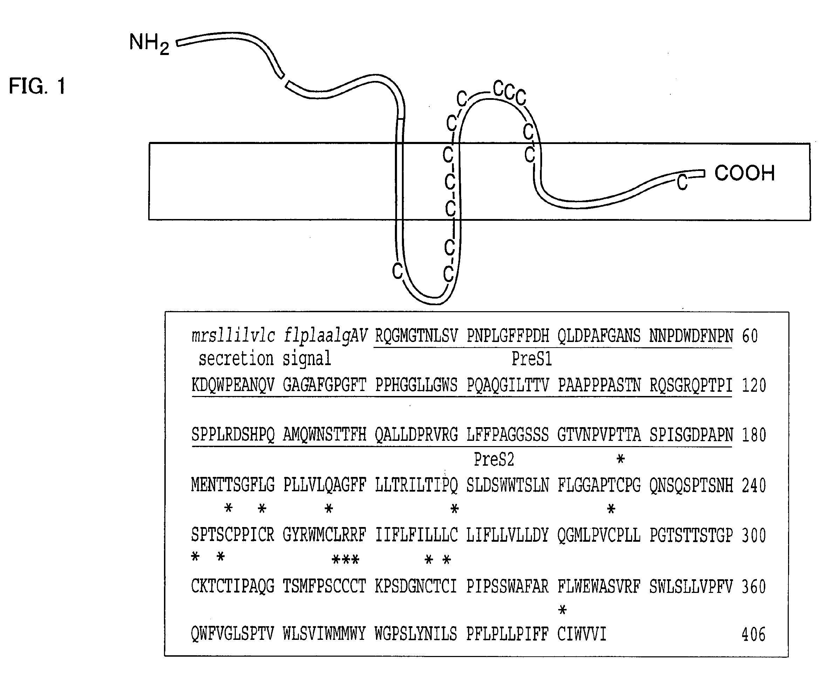

The site of modified Cys residue is not particularly limited as long as the functionality of the HBsAg particles is maintained. The HBsAg L protein includes PreS1, PreS2, and S protein, wherein the PreS1 and PreS2 serve as hepatocytes recognition sites. It is believed that modification of amino acid in the S protein has only a small effect on the functionality of the HBsAg particles. It is therefore possible, by modifying some of the 14 cysteine (Cys) residues in the S protein (represented by "C" in the schematic diagram of Fig. 1), to break the disulfide bond without losing the ability of the HBsAg particles to recognize hepatocytes or form particles.

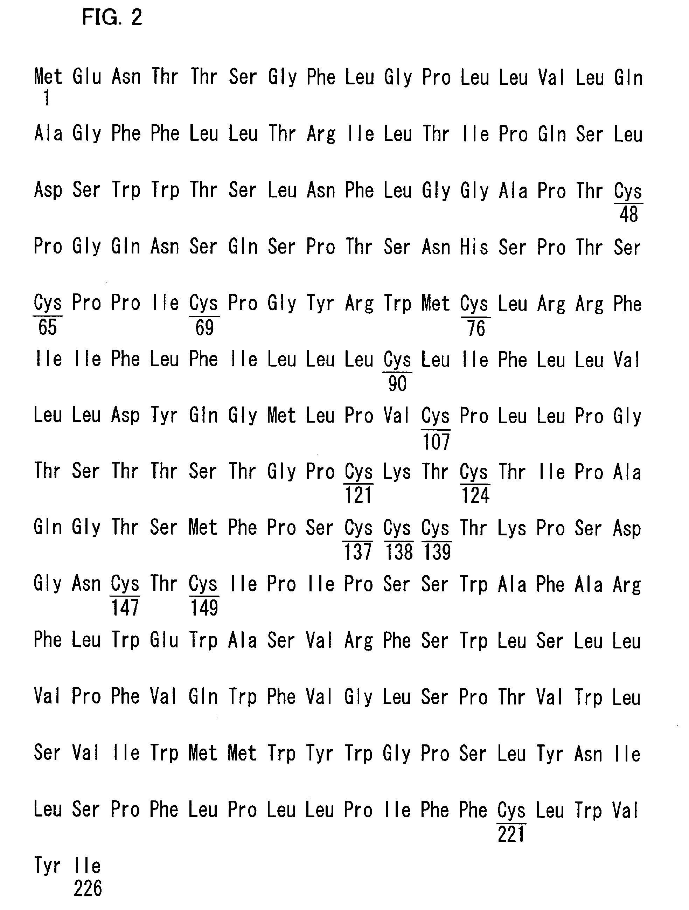

The 14 Cys residues in the S protein are amino acid residues 48, 65, 69, 76, 90, 107, 121, 124, 137, 138, 139, 147, 149, and 221 from the N-terminus of the amino acid sequence of the S protein (underlined in Fig. 2). As illustrated in the schematic representation in Fig. 1, the HBsAg protein is a transmembrane protein with its PreS region at the N-terminus sticking out of the particle. The PreS region is contiguous to an S protein portion that extends through the particle membrane, reenters the membrane inside the particle, and comes out of the membrane outside of the particle and enters the membrane again with the C-terminus buried inside the membrane. That is, the S protein exists inside and outside of the particle with three transmembrane sites. It is believed that the 14 Cys residues are scattered in these regions, with Cys residues 48, 65, and 69 inside the particles, Cys residues 76, 90, 107 in the second transmembrane region, Cys residues 121, 124, 137, 138, 139, 147 exposed outside of the particles, and Cys residues 149 and 221 in the third transmembrane region.

Any of the 14 Cys residues may be modified but those in the transmembrane sites and outside of the particles, near the C-terminus, are preferable. Specifically, substitution of Cys residues 76, 90, 137, 138, 139, 147, 149, and 221 are preferable, and substitution of two or more of these 8 Cys residues is more preferable. Further, substitution of all of the 8 Cys residues, or substitution of the 8 Cys residues except for Cys residue 137 or 138 is particularly preferable.

The Cys residues may be replaced by any amino acid, but a hydrophobic amino acid is preferable because the amino acids in the transmembrane sites exist in the hydrophobic lipid bilayer membrane of the particle. Hydrophobic amino acids include alanine, glycine, isoleucine, leucine, methionine, phenylalanine, proline, tryptophane, tyrosine, and valine, among which substitution by an alanine residue is preferable. On the other hand, for the Cys residues believed to exist outside and inside the particles, substitution by a hydrophilic amino acid is preferable. Hydrophilic amino acids include arginine, asparagine, aspartic acid, glutamic acid, glutamine, histidine, lysine, serine, and threonine, among which substitution by a serine residue is preferable.

The Cys residues can be modified by a common site-specific mutation introducing method. For example, a mutant protein may be prepared by introducing point mutation in the base sequence using PCR, or a site-specific mutation inducing method (Hashimoto-Gotoh, Gene 152, 271-275 (1995), elsewhere) may be used. Further, a method described in Cell Technology, separate volume, *New Cell Technology Experiment Protocol, Shujunsha*, 241-248 (1993), or a method using a commercially available kit (for example, Quickchange Site-Directed Mutagenesis Kit, Stratagene) may be used as well.

Among these methods, a method of introducing mutation using PCR is particularly preferable. Specifically, the method prepares a primer into which mutation has been introduced, and the primer is hybridized with a base sequence coding for a Cys residue to be replaced. The hybridized fragments are then amplified by PCR. In this manner, the method amplifies a gene that encodes an L protein in which the Cys residue is replaced with a different amino acid. Expression of the gene for example in eukaryotes produces hollow nanoparticles in which the Cys residue has been replaced.

The receptor on the surface of the resulting particles may be modified to any bio-recognizing molecule. This enables hollow nanoparticles of the present invention to very specifically deliver and transfer substances to any cell or tissue, including the hepatocytes.

The particle-forming protein is not just limited to the hepatitis B virus surface-antigen protein as long as it can form particles. For example, natural proteins derived from animal cells, plant cells, viruses, or fungi may be used. Various types of synthetic proteins may be used as well. Further, when there is a possibility that, for example, virus-derived antigen proteins may trigger antibody reaction in a target organism, a particle-forming protein with suppressed antigenic action may be used. For example, such a protein may be hepatitis B virus surface-antigen protein modified to suppress its antigenic action, or other types of modified proteins (hepatitis B virus surface-antigen protein modified by genetic engineering), as disclosed in International Publication WO01/64930. Further, for the particle-forming protein, the hepatitis B virus surface-antigen protein or modified hepatitis B virus surface-antigen protein may be joined to other proteins such as a growth factor or antibody.

The bio-recognizing molecule incorporated in the particle-forming protein (may be contained in the particle-forming protein itself, or fused with the particle-forming protein by being ligated either directly or indirectly) may be, for example, cell function regulatory molecules such as a growth factor or cytokine; molecules, such as a receptor, cell surface-antigen, or tissue specific antigen, for recognizing cells or tissues; molecules derived from viruses or micro organisms; antibodies; sugar chains; and lipids. Specific examples include a cancer-specific antibody for the EGF receptor or IL-2 receptor, and EGF. Receptors displayed on the HBV are another example. These molecules are suitably selected according to the type of target cell or tissue. As the term is used herein, the "bio-recognizing molecule" refers to molecules that recognize specific cells. (In other words, molecules that render the hollow nanoparticles the ability to recognize specific cells.)

The hollow nanoparticles of the present invention so prepared are useful in specifically transporting a cell transfer substance to a specific cell. For example, hollow nanoparticles of the present invention may be particles of hepatitis B virus surface-antigen protein, and may be administered through intravenous injection as a drug containing a cell transfer substance inside the hollow nanoparticles. The particles circulate through the body and reach the hepatocytes by the action of the hepatocyte specific receptors displayed on the particle surface, thereby infecting the host. The cell transfer substance is then transported into the hepatocytes, thereby specifically transferring the cell transfer substance into the liver tissue.

The cell transfer substance encapsulated in the hollow protein nanoparticles is not particularly limited. For example, the cell transfer substance may be genes such as DNA or RNA; natural or synthetic proteins; oligonucleotides; peptides; medicaments; or natural or synthetic compounds.

These cell transfer substances may be incorporated into the hollow nanoparticles by various methods commonly used in chemical or molecular biological experimental techniques. Some of the preferred examples include an electroporation method, ultrasonic method, simple diffusion method, and a method using charged lipids. When the cell transfer substance is a protein, the particle-forming protein may be fused with the cell transfer substance to form the particles. When applying the method, the particle-forming protein is fused with the cell transfer substance in the manner described below. For example, a plasmid is prepared that has incorporated a gene encoding a hepatitis B virus surface-antigen protein. Downstream of the gene, the plasmid also includes a gene encoding a protein drug. The plasmid is used to produce particles in a eukaryotic cell, thereby producing a drug in which the protein drug is fused with the hepatitis B virus surface-antigen protein forming the particles.

Other than intravenous injection, the drug may be administered through oral administration, intramuscular administration, intraperitoneal administration, subcutaneous administration, or other administration routes.

The drug of the present invention allows a substance to be specifically transported into cells or tissues *in vivo* or *in vitro*. Specific transport of a substance into a specific cell or specific tissue with the use of the drug may be used as a treatment method of various diseases, or one of the steps in the procedure of the treatment method.

In the following, the present invention will be described in more detail by way of Examples with reference to the attached drawings. It should be appreciated that the present invention is not limited in any way by the following Examples, and various modifications to details of the invention are possible.

### (Examples)

In the following, HBsAg refers to hepatitis B virus surface antigen. HBsAg is an envelope protein of HBV, and includes an S protein consisting of 226 amino acids, as shown in Fig. 2. M protein includes the entire sequence of the S protein with additional 55 amino acids (pre-S2 peptide) at the N-terminus. L protein contains the entire sequence of the M protein with additional 108 or 119 amino acids (pre-S1 peptide) at the N-terminus.

The pre-S regions (pre-S1, pre-S2) of the HBsAg L protein have important roles in the binding of HBV to the hepatocytes. The Pre-S1 region has a direct binding site for the hepatocytes, and the pre-S2 region has a polymeric albumin receptor that binds to the hepatocytes via polymeric albumin in the blood.

Expression of HBsAg in the eukaryotic cell causes the protein to accumulate as a membrane protein on the membrane surface of the endoplasmic reticulum. The L protein molecules of HBsAg agglomerate and are released as particles into the ER lumen, carrying the ER membrane with them as they develop.

The Examples below used HBsAg L protein.

### (Example 1) Preparation of a gene plasmid encoding HBsAg L protein with substituted Cys residue

According to the method described in J. Biotechnol., Vol. 33:, No. 2, 157-174, 1994 reported by the inventors of the present invention, a gene fragment encoding a HBsAg L protein incorporated in pGLDL IIP39-RcT was inserted in an animal cell expression plasmid pTB1455, downstream of the SRα' promoter. As a result, a pBO442 plasmid was obtained. The base sequence of the HBsAg L protein is represented by SEQ ID NO: 1, and the amino acid sequence is represented by SEQ ID NO: 2. The pBO442 plasmid was used to transform *Escherichia coli* K12 strain (DH 5α or XL-1 Blue) for methylating DNA, and methylated pBO442 plasmid was purified.

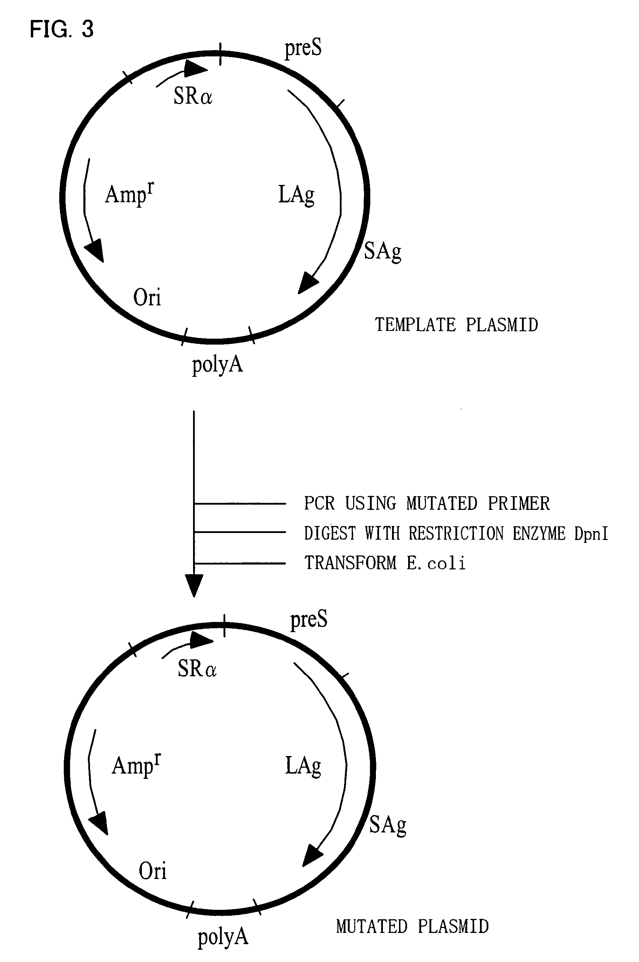

In a region of the pBO442 plasmid encoding the HBsAg L protein, a base sequence coding for a Cys residue was modified to a base sequence coding for an Ala residue or Ser residue. This was achieved by introducing mutation by a site-specific mutation introducing method using PCR, using the pBO442 plasmid as a template. Referring to Fig. 3, the following specifically describes a preparation method of a HBsAg L protein gene plasmid in which Cys residue 48 is replaced by a Ser residue, for example.

As shown in Fig. 3, the pBO442 plasmid as a template plasmid has an SRα' promoter. Downstream of the SRα' promoter is a coding region for the HBsAg L protein. A region of the plasmid including a codon (tgt) coding for Cys residue 48 of the HBsAg L protein was hybridized with a mutated synthetic oligonucleotide (primer). Using the plasmid as a template, the strand was extended by PCR. Here, the mutated primer is designed to cause a mismatch with the codon for the Cys residue 48 of the template (tgt for the template, aga for the primer). This allows for amplification of the plasmid in which a codon (tgt) for Cys residue 48 is replaced with a Ser codon (tct).

Table 1 shows base sequences of mismatch primers prepared in the manner described above, wherein the primers in the mismatch primer set (rows 1 through 14) are for substituting the 14 Cys residues, respectively, and the primers in the mismatch primer set (rows 15 through 17) are for substituting two or three adjacent Cys residues. The substitution position indicates the position of substituted Cys residue from the N-terminus of the amino acid sequence in the S protein. Table 1 also shows the size and sequence of the corresponding primer (a pair of sense and anti-sense strands), and annealing temperatures in the PCR reaction. The substitution position is denoted, for example, by the notation "C/48/S", which means that Cys residue 48 has been replaced with a Ser residue. Similarly, "C/76/A" means that Cys residue 76 has been replaced with an Ala residue.

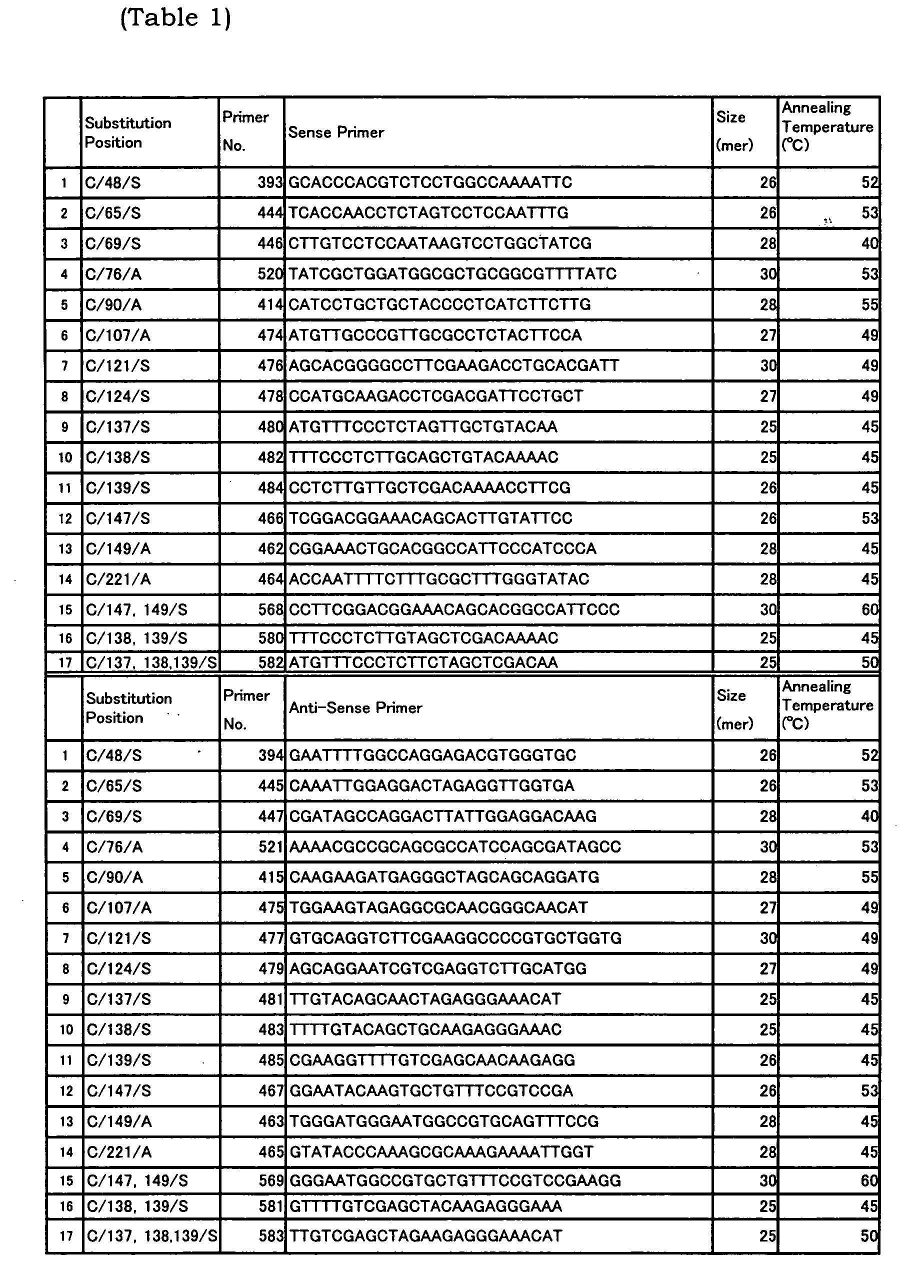

The base sequences of the 17 pairs of 34 primers are respectively represented by SEQ ID NOs: 3 through 36 in the order of appearance in Table 1. Specifically, SEQ ID NOs: 1 and 2 represent base sequences of the primer set for substituting Cys residue 48. Similarly, the base sequences of the primer sets for substituting Cys residues 65, 69, 76, 90, 107, 121, 124, 137, 138, 139, 147, 149, and 221 are represented by SEQ ID NOs: 3 and 4, 5 and 6, 7 and 8, 9 and 10, 11 and 12, 13 and 14, 15 and 16, 17 and 18, 19 and 20, 21 and 22, 23 and 24, 25 and 26, and 27 and 28, respectively. Further, SEQ ID NOs: 29 and 30 represent base sequences of the primer set for substituting Cys residues 147 and 149. Similarly, SEQ ID NOs: 31 and 32 represent base sequences of the primer set for substituting Cys residues 138 and 139. The base sequences of the primer set for substituting Cys residues 137, 138, and 139 are represented by SEQ ID NOs: 33 and 34.

The PCR was carried out in a 50 µl solution containing 50 nmol template DNA, 15 pmol synthetic DNA primer (each mutated primer), 40 nmol dATP, 40 nmol dCTP, 40 nmol dGTP, 40 nmol dTTP, 2.5 unit Pfu turbo DNA polymerase, 10 mM KCl, 10 mM (NH₄)₂SO₄, 20 mM Tris-Cl (pH 8.75), 2 mM MgSO₄, 0.1% Triton® X-100, and 100 µg/ml BSA. After 30-second heating at 95°C, the reaction was carried out in 18 cycles at 95°C for 30 seconds and at 68°C for 20 minutes with 1-minute annealing in between. Different annealing temperatures were used for the different primers, as shown in Table 1.

After the reaction, 1 µl of restriction enzyme DpnI (10,000 unit/ml) for digesting methylated DNA was added. The mixture was allowed to react for 1 hour at 37°C. As described above, the template plasmid DNA with unsubstituted Cys residue is obtained by transforming *E. coli* that methylates DNA. Accordingly, the template DNA is methylated. On the other hand, DNA with substituted Cys residue is not methylated. Therefore, the restriction enzyme DpnI only digests the template DNA. The remaining DNA, i.e., the L protein DNA with substituted Cys residue was used to transform *E. coli* XL-1 Blue, and plasmid DNA was extracted from the resulting colonies. From the restriction enzyme map and the base sequence, it was confirmed that the resulting plasmid DNA was successfully mutated.

The L protein plasmid with substituted Cys residue was used as a template, and the PCR reaction was repeated using another mismatch primer. In this manner, an L protein gene plasmid with increased numbers of substituted Cys residues was prepared.

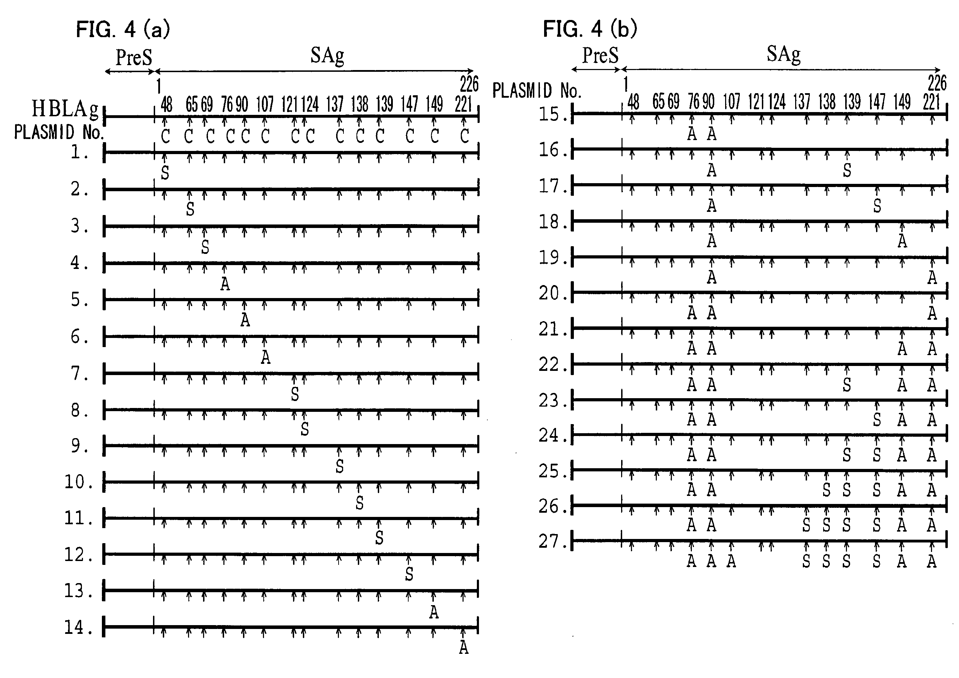

According to these procedures, a total of 27 plasmids with 1 to 9 modified Cys residues were prepared. Table 2 shows these 27 plasmids (numbered 1 to 27), their names (pBO***) and substitution positions in the expressed proteins. Fig. 4 schematically illustrates the primary structure of the HBsAg L proteins. The diagram also shows positions of cysteine in the S protein (indicated by arrows), and substitution positions of amino acid (S (serine) or A (alanine)) in the S protein encoded by the gene in each plasmid.

**(Table 2)**

| Plasmid | Plasmid Name | Substitution Position |
|---|---|---|
| No. | pBO No. | |
| 1 | 454 | C/48/S |
| 2 | 497 | C/65/S |
| 3 | 498 | C/69/S |
| 4 | 468 | C/76/A |
| 5 | 455 | C/90/A |
| 6 | 456 | C/107/A |
| 7 | 460 | C/121/S |
| 8 | 461 | C/124/S |
| 9 | 465 | C/137/S |
| 10 | 466 | C/138/S |
| 11 | 467 | C/139/S |
| 12 | 499 | C/147/S |
| 13 | 469 | C/149/A |
| 14 | 470 | C/221/A |
| 15 | 511 | C/76,90/A |
| 16 | 514 | C/90/A,C/139/S |
| 17 | 518 | C/90/A, C/147/S |
| 18 | 513 | C/90,149/A |
| 19 | 512 | C/90,221/A |
| 20 | 519 | C/76,90,221/A |
| 21 | 520 | C/76,90,149,221/A |
| 22 | 528 | C/76,90,149,221/A, C/139/S |
| 23 | 529 | C/76,90,149,221/A, C/147/S |
| 24 | 533 | C/76,90,149,221/A, C/139,147/S |
| 25 | 539 | C/76,90,149,221/A, C/138,139,147/S |
| 26 | 540 | C/76,90,149,221/A, C/137,138,139,147/S |
| 27 | 541 | C/76,90,149,221/A, C/107,137,138,139,147/S |

### (Example 2) Expression of mutated HBsAg particles in COS7 cells and detection of the particles

### (1) Expression of mutated HBsAg particles in COS7 cells

Cell line COS7 derived from the monkey kidney was cultured in a Dulbecco-modified eagle medium (DMEM) containing 5% fetal bovine serum (FBS). The incubation was made at 37°C and in the presence of 5% CO₂. For each sheet of T-75 flask (product of Falcon), 4 × 106 cells were obtained. Meanwhile, 18.5 mg of glucose was added to 10 M1 of RPMI1640 medium. To 1 M1 of the solution was added 2 µl of 50 mM dithiothreitol (DTT). In 0.3 M1 of the solution, 4 × 106 COS7 cells and 5 µg of the plasmid DNA obtained in Example 1 were suspended. The solution was moved to an electroporation cuvette (4 mm across electrodes), and gene transfer was carried out with an electroporator (Bio-Rad) at 950 µF and 0.3 kV. The cells in the cuvette were moved to a 60 mm dish (product of Falcon), and were cultured in 6 M1 of DMEM containing 5% FBS. The incubation was made for 14 to 15 hours at 37°C in the presence of 5% CO₂. By changing the medium to 6 M1 serum-free medium CHO-SFM II (Invitrogen), the cells were further incubated for 4 days, and the medium was collected.

### (2) Detection of mutated HBsAg particles based on antigenicity

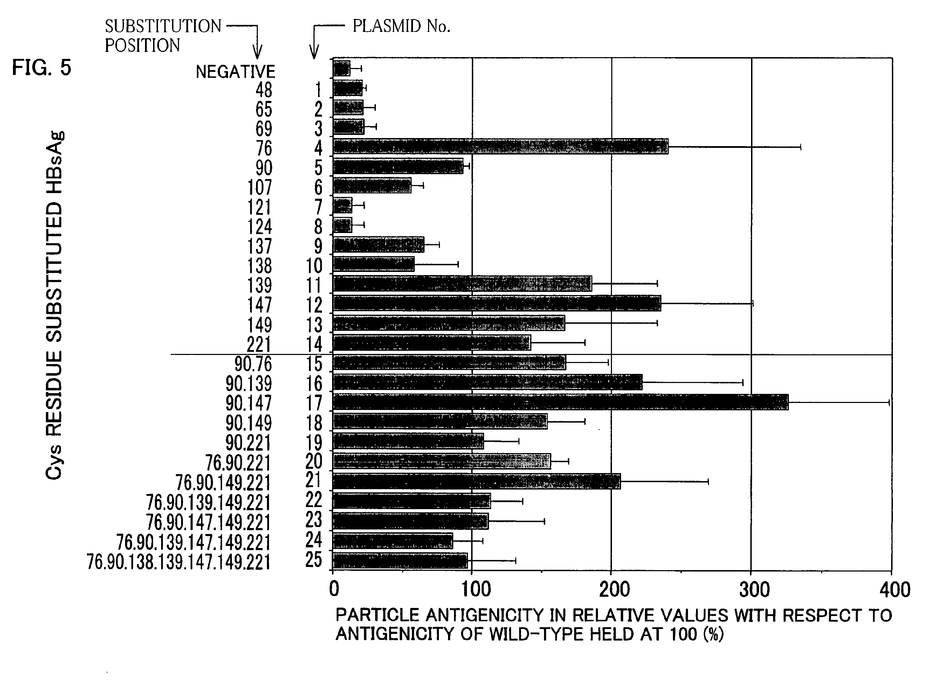

To 90 µl of collected medium was added 90 µl of Dulbecco phosphate buffer (PBS) containing 1% FBS, and antigenicity was detected with the IMX HBsAg assay system (Dinabot). The presence of antigenicity was regarded as the formation of HBsAg particles. Therefore, the presence of HBsAg particles in the culture medium was confirmed when antigenicity was detected. Fig. 5 shows the result of detection for the expression of 25 mutant HBsAg that were respectively mutated by the plasmids 1 through 25. Antigenicity was measured in relative values with respect to that of the wild-type held at 100. The result of detection for the culture medium in which no plasmid was used and no particles were formed by the transformation was a negative control. Note that, for the experiments that were carried out three or more times, the standard deviation is indicated by bars.

The result of single amino acid substitution for the 14 Cys residues (rows 1 to 14) showed that those with the substitution of Cys residues in the transmembrane regions had desirable antigenicity. In particular, those with the substitution of Cys residues 76, 90, 149, and 221 had antigenicity that compared to or excelled that of the wild-type. Further, those with the substitution of Cys residues outside of the particles and near the C-terminus maintained desirable antigenicity. In particular, those with the substitution of Cys residues 139 and 147 had antigenicity that compared to or excelled that of the wild-type. By combining these Cys residue substitution positions that incur particularly desirable antigenicity, HBsAg L proteins were prepared in which 2 to 9 Cys residues had been replaced (rows 15 to 25). All of these HBsAg L proteins had antigenicity that compared to or excelled that of the wild-type.

### (3) Detection of mutated HBsAg particles by Western blotting

The molecular weight of mutant particles and the presence or absence of dimerized particles were examined by sodium dodecyl sulfate-polyacrylamide gel electrophoresis (SDS-PAGE) and Western blotting.

Specifically, to 180 µl of collected medium was added 60 µl of anti-HBs mouse monoclonal antibody-immobilized microparticles of the IMX HBsAg assay system. The mixture was allowed to stand overnight at 4°C with the tube slowly rotated. The particles in the medium were sedimented with the microparticles for 3 minutes, using a desktop high-speed centrifuge. The resulting sediments were suspended in 0.5 M1 TBST (10mM Tris-HCl buffer, pH 7.5, 150 mM NaCl, 0.1 Tween20). The washing was carried out 5 times by repeating the procedure of centrifugation and sediment collection.

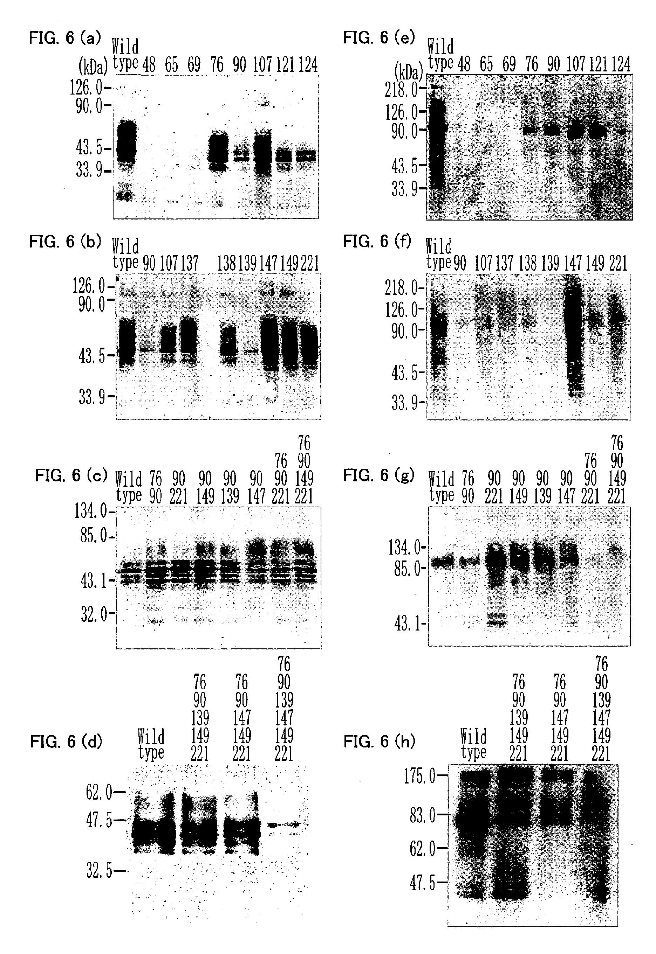

The sediment was separated in half and subjected to SDS-PAGE under reduced condition and non-reduced condition, respectively. Under reduced condition, SDS-PAGE was performed after treating the sediment in mercaptoethanol at 95°C for 5 minutes. The process prevented formation of disulfide bonds between Cys residues, allowing the proteins to be detected without causing polymerization. On the other hand, under non-reduced condition, SDS-PAGE was performed without heat-treating the sediment in mercaptoethanol. Accordingly, proteins were detected in polymerized form. Western blotting was carried out on the electrophorased gel, using an anti-HBs goat antibody-biotin conjugation of the IMX HBsAg assay system as the first antibody, and an alkaliphosphatase-labeled anti-biotin rabbit antibody as the secondary antibody. (Fig. 6(a) through Fig. 6(h)). Fig. 6(a) through Fig. 6(d) show results under reduced condition, and Fig. 6(e) through Fig. 6(h) show results under non-reduced condition. Numbers on the lanes indicate substitution positions of Cys residues. As can be seen from the results, all mutant HBsAg particles showed bands, near 53 kDa range under reduced condition, and near 100 kDa range under non-reduced condition. This confirmed that the mutant HBsAg had a molecular weight of 53 kDa, and that the mutant HBsAg existed as dimmers when in particle form. It is believed that the presence of the HBsAg dimmers is related to the ability of the protein to form particles.

### (Example 3) Transfer of gene into HepG2 cell using mutant HBsAg particles

A gene fragment encoding the HBsAg L protein incorporated in the pGLDL II P39-RcT was replaced with the mutant HBsAg gene prepared in Example 1, so as to prepare a mutant HBsAg gene plasmid for expression in yeasts. The mutant HBsAg gene plasmid was expressed in yeasts (particles were prepared) according to the method disclosed in International Publication WO01/64930.

The following describes how the mutant HBsAg particles are used to transfer the GFP gene into the human hepatic cancer cell HepG2.

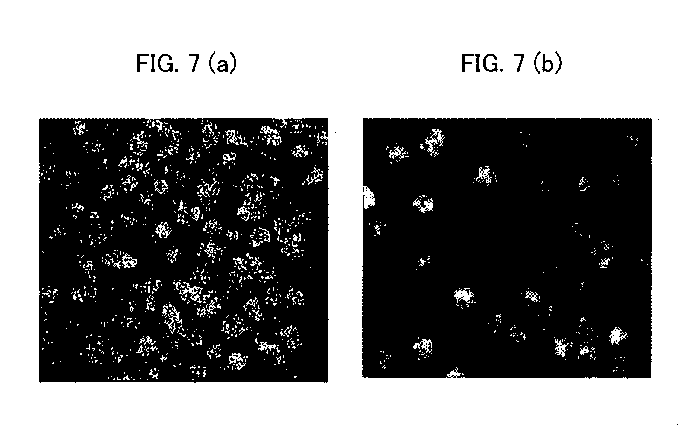

First, the human hepatic cancer cells HepG2 were inoculated on a 3.5 cm glass-bottom Petri dish with 1 × 10⁵ cells/well. The cells were incubated overnight using D-MEM containing 10% fetal bovine serum. The incubation was carried out at 37°C in the presence of 5% CO₂ until the exponential growth stage was reached. The mutant HBsAg particles were mixed with a green fluorescent protein expression plasmid (GFP expression plasmid pTB701-hGFP), and the GFP expression plasmid was encapsulated by carrying out electroporation under the conditions of 110 V and 950 µF, using a 4 mm-pitch cuvette. The HBsAg particles containing the GFP expression plasmid were mixed with a HepG2 culture medium, and were incubated for 4 days in D-MEM at 37°C in the presence of 5% CO₂. GFP expression in the HepG2 was observed with a confocal laser fluorescence microscope (Fig. 7(a)). As a comparative example, a sample was also prepared for the wild-type HBsAg particles. According to the method described above, substantially the same number of HepG2 cells were cultured in a Petri dish, and particles containing an equal amount of GFP gene plasmid were mixed with an equal amount of the HepG2 culture medium. The sample was incubated for 4 days in D-MEM at 37°C in the presence of 5% CO₂. GFP expression in the HepG2 was observed with a confocal laser fluorescence microscope (Fig. 7(b)).

It can be seen from Fig. 7 that more cells fluoresced when the mutant HBsAg was used for the gene transfer. That is, by mutating the protein forming the HBsAg particles, the efficiency of gene transfer to the cell was improved.

The experiment showed that, on the cultured cell level, the HBsAg particles with the substituted Cys residue can be used to transfer genes specifically to the human hepatocytes with improved efficiency.

### (Example 4) Gene transfer to HepG2 cells using mutant HBsAg particles containing a mutant gene with 7 or 8 mutated Cys residues

Gene transfer to the HepG2 cells was carried out using a mutant HBsAg gene with 7 to 8 cysteine substitutions. As the mutant HBsAg genes, three kinds of plasmids prepared from the wild-type HBsAg genes were used: Plasmid pBO539 with Cys residues 76, 90, 149, 221 substituted to Ala residues, and Cys residues 138, 139, and 147 substituted to Ser residues; plasmid pBO552 with Cys residues 76, 90, 149, 221 substituted to Ala residues, and Cys residues 137, 139, 147 substituted to Ser residues; and plasmid pBO540 with Cys residues 76, 90, 149, 221 substituted to Ala residues, and Cys residues 137, 138, 139, 147 substituted to Ser residues (Table 3). The plasmids pBO539, pBO552, and pBO540 were used to prepare HBsAg particles BNP-Lm7a, BNP-Lm7b, and BNP-Lm8, respectively.

**(Table 3)**

| Plasmid | Expressed Particles | Substitution Position |
|---|---|---|
| pBO539 | BNP-Lm7a | C/76,90,149,221/A C/138,139,147/S |
| pBO552 | BNP-Lm7b | C/76,90,149,221/A C/137,139,147/S |
| pB0540 | BNP-Lm8 | C/76,90,149,221/A C/137,138,139,147/S |

### (1) Preparation of mutant HBsAg particles

In order to obtain mutant HBsAg particles, mutant HBsAg particle expression vector DNA was transferred into COS7 cells by an electroporation method. After culturing the cells for 4 days, the supernatant was collected, concentrated by centrifugal filtration, and the particles were collected. The following specifically describes the procedure used in the experiment.

For each sample, COS7 cells were cultured in a 5% FCS-MEM medium to obtain 80% confluent cells. On a new medium, the cells were treated with trypsin after 6 to 8 hours and were collected and counted with a hemocytometer. After counting, the cells were fractionated and centrifuged to obtain 4 × 10⁶ cells in each 1.5 ml tube. The centrifugation was carried out for 30 seconds at 12000 rpm. After centrifugation, the supernatant was suctioned and removed, and the cells were suspended in 300 µl of electroporation solution (Epmedium: RPMI-1640 + 10 mM glucose + 0.1 mM DDT).

To the suspended solution of cells was added 5 µg of the plasmid DNA pBO539, pBO552, or pBO540 shown in Table 3. The solution was then moved to a cuvette (4 mm gap) that had been pre-cooled on ice. Immediately after a voltage was applied under 0.3 KV and 950 µF (electroporation), the sample was ice-cooled. Thereafter, the cells in the cuvette were re-suspended in an 8 ml medium, and were inoculated in two 6 cm Petri dishes in equal amounts.

After culturing the cells at 37°C for 14 to 15 hours, the medium in each 6 cm Petri dish was changed to 3 ml of CHO-S-SFM II (GIBCO). The cells were further incubated for 4 days on the new medium. After 4 days, the cells and supernatant were separately collected, and were stored at -20°C and 4°C, respectively. For each plasmid DNA sample, electroporation was carried out 4 times.

### (2) Measurement of particle concentration using IMxHBsAg assay system

The supernatant of the cultured cells was used for the IMx analysis as described below.

The IMxHBsAg assay system (Dinabot) is a fully automated immunoassay system that detects HBs antigens in the supernatant, using EIA employing a sandwich method. The antigenicity so detected in the supernatant is represented by RATE value. As required, the antigenicity was compared with the antigenicity of a standard HBsAg (human serum-derived HBsAg particles) provided in the system, so as to convert the concentration of the expressed particles to a HBsAg equivalent. The measured sample was 90 µl of supernatant mixed with 90 µl of 1% fetal bovine serum-containing Dulbecco phosphate buffer saline solution (diluent). The measurement was carried out always with a positive control (reference HBsAg) and a negative control (only diluent), and a HBsAg equivalent was calculated based on a calibration curve. A comparison was also made with the supernatant of the COS7 cells that have incorporated the wild-type HBsAg gene vector (pB0411). The results are shown in Table 4 below.

**(Table 4)**

| Sample | RATE | Particle Concentration (ng/ml) |
|---|---|---|
| Positive Control | 201.1 | - |
| Nagative Control | 3.6 | - |
| Wild-Type HBsAg Particles | 94.7 | 132.9 |
| BNP-Lm7a | 15.7 | 17.7 |
| BNP-Lm7b | 15.4 | 17.2 |
| BNP-Lm8 | 10.6 | 10.2 |

It can be seen from the result that the standard HBsAg equivalents (RATE values) of the particles are higher than that of the negative control, yielding values of 15.7, 15.4, and 10.6 for the BNP-Lm7a, BNP-Lm7b, and BNP-Lm8, respectively. Therefore, it can be said that all samples had mutant HBsAg proteins secreted and expressed in the supernatant.

### (3) Concentration by ultra centrifugal filtration concentration method

The particle concentration and the level of particle expression for the Cys-substituted particles are low in the IMxHBsAg assay system, requiring the particles to be concentrated for actual use. As such, the particles were concentrated by ultra centrifugal filtration concentration, as described below.

Sixteen ml of the supernatant was transferred to a centrifugal filtration concentration device (Viva spin, 1,000,000 MWCO, Sartorius), and concentrated to a liquid amount of 800 µl at 4°C and 3500 rpm for 45 minutes. Ninety µl out of the 800 µl liquid was used to measure a HBsAg equivalent by IMx. From the amount of antigen calculated from the calibration curve, the yield was determined to be around 40%.

### (4) Measurement of S antigenicity of L, S coexpression particles by IMx

COS7 cells were cultured in a 5% FCS-MEM medium to obtain 80% confluent cells. The cells were treated with trypsin after 6 to 8 hour incubation on a new medium, and were collected and counted with a hemocytometer. After counting, the cells were fractionated and centrifuged to obtain 4 × 10⁶ cells in each 1.5 ml tube. The centrifugation was carried out for 30 seconds at 12000 rpm. After centrifugation, the supernatant was suctioned and removed, and the cells were suspended in 300 µl of electroporation solution (Ep medium: RPMI-1640 + 10 mM glucose + 0.1 mM DDT).

To the suspension solution of the cells were added 5 µg of wild-type L-particle (particles of HBsAg L protein) expression plasmid DNA (pB0441) or cysteine-modified L-particle expression plasmid DNA, and 1 µg of wild-type S-particle (particles of HBsAg S protein) expression plasmid DNA (pB603) or cysteine-modified S-particle expression plasmid DNA. The mixture was transferred to a cuvette (4 mm gap). Immediately after a voltage was applied under 0.3 KV and 950 µF (electroporation), the sample was ice-cooled. Thereafter, the cells in the cuvette were re-suspended in an 8 ml medium, and were inoculated in two 6 cm Petri dishes in equal amounts.

After culturing the cells at 37°C for 14 to 15 hours, the medium in each 6 cm Petri dish was changed to 3 ml of CHO-S-SFM II (GIBCO). The cells were further incubated for 4 days on the new medium. After 4 days, the cells and supernatant were separately collected, and were stored at -20°C and 4°C, respectively. With a IMx HBsAg assay system, a particle concentration (HBsAg equivalent) of the supernatant was measured.

**(Table 5)**

| Sample | RATE |
|---|---|
| Positive Control | 255.0 |
| Nagative Control | 6.2 |
| Wild-Type HBsAg Particles | 363.9 |
| BNP-Lm7b | 58 |
| BNP-Lm8 | 47.6 |

It can be seen from the result that the HBsAg equivalents (RATE values) of the mutant HBsAg particles are considerably higher than those obtained in Experiment (2), yielding values of 58.0 and 47.6 for the BNP-Lm7b and BNP-Lm8, respectively.

### (5) DNA transfer into mutant HBsAg particles

Five hundred ng of GFP expression vector DNA was added to the concentrated mutant HBsAg particles (BNP-Lm8) obtained in (3) above, and the total amount was adjusted to 500 µl. In a 4-mm gap cuvette for electroporation, the sample was electrophorased at 50 V and 750 µF and allowed to stand for 5 minutes at room temperature. As a result, mutant HBsAg particles were prepared that has incorporated GFP expression vector DNA.

### (6) Gene transfer into HepG2 cells using L particles that have incorporated GFP expression vector DNA

HepG2 cells in a 6 cm Petri dish were treated with trypsin, and collected cells were counted with a hemocytometer. After confirming the cell count, the cells were fractionated on an 8-well chamber slide, with each well containing 9000 cells. The cells were incubated overnight at 37°C. To the HepG2 cells were added mutant HBsAg particles (GFP-BNP-Lm8) that have incorporated the GFP expression vector obtained in (5), and wild-type HBsAg particles that have incorporated GFP expression vector DNA. As a positive control, a mixture of the HepG2 cells and a compound material containing the GFP expression vector DNA and FuGene 6 (Roche) was prepared. As a negative control, a mixture of the HepG2 cells and an electroporation buffer containing neither the particles nor vector, or a mixture of the HepG2 cells and only the GFP expression vector DNA was prepared. After 3 days, cell fluorescence was observed with a confocal microscope. Note that, the HBsAg particles enveloping the GFP expression vector DNA contained the GFP expression vector in various proportions.

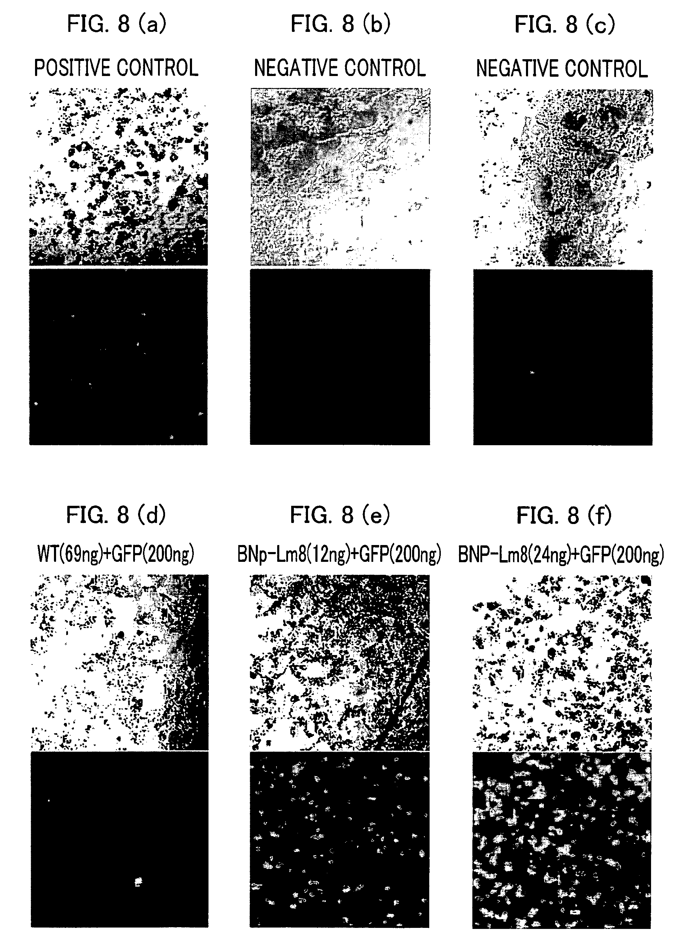

Fig. 8 represents micrographs in confocal microscopy, showing transmission images in the upper column, and fluorescent images in the lower column. In Fig. 8, (a) represents a positive control, (b) represents a negative control using only the electroporation buffer without particles or vector, (c) represents a negative control using only GFP expression vector DNA, (d) represents a sample containing the wild-type HBsAg particles (HBsAg particles (69 ng)) + GFP expression vector DNA (200 ng), (e) represents a sample containing GFP-BNP-Lm8 particles (BNP-Lm8 (12 ng)) + GFP expression vector (200 ng)), and (f) represents a sample containing GFP-BNP-Lm8 particles (BNP-Lm8 (24 ng)) + GFP expression vector (200 ng).

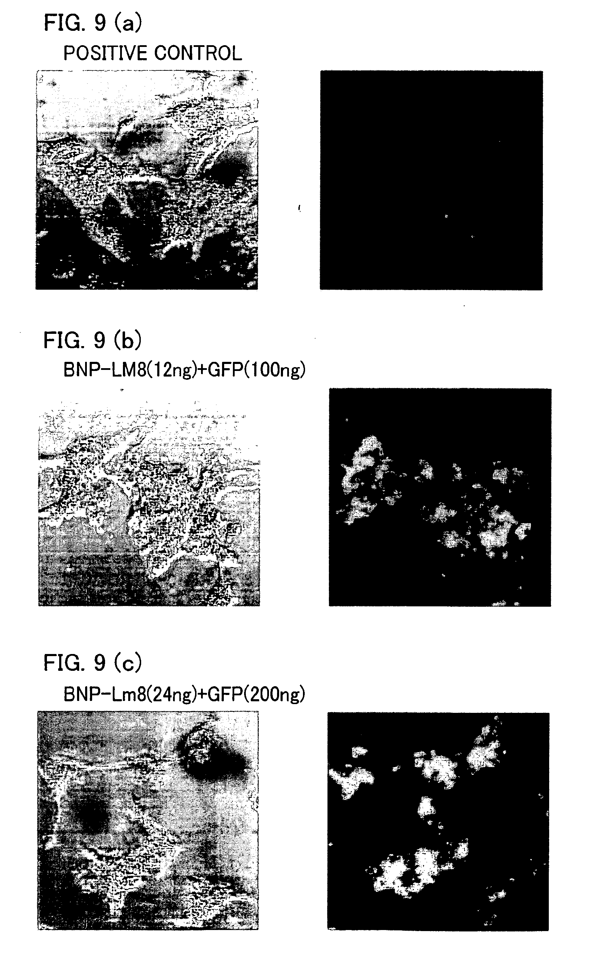

Fig. 9(a) through Fig. 9(c) are enlarged views of Fig. 8, magnifying Fig. 8(a), Fig. 8(e), and Fig. (f), respectively.

It can be seen that the cells fluoresce green far more strongly in the sample containing the GFP-BNP-Lm8 particles than in the sample containing the wild-type particles enveloping the GFP expression vector. In fact, the sample containing the GFP-BNP-Lm8 particles showed fluorescence even when the amount of particles added was as small as 10 ng. By repeating the experiment, the DNA transfer efficiency of the BNP-Lm8 particles into the cells was confirmed to be reproducible.

### (7) Preservability of BNP-LM8 particles

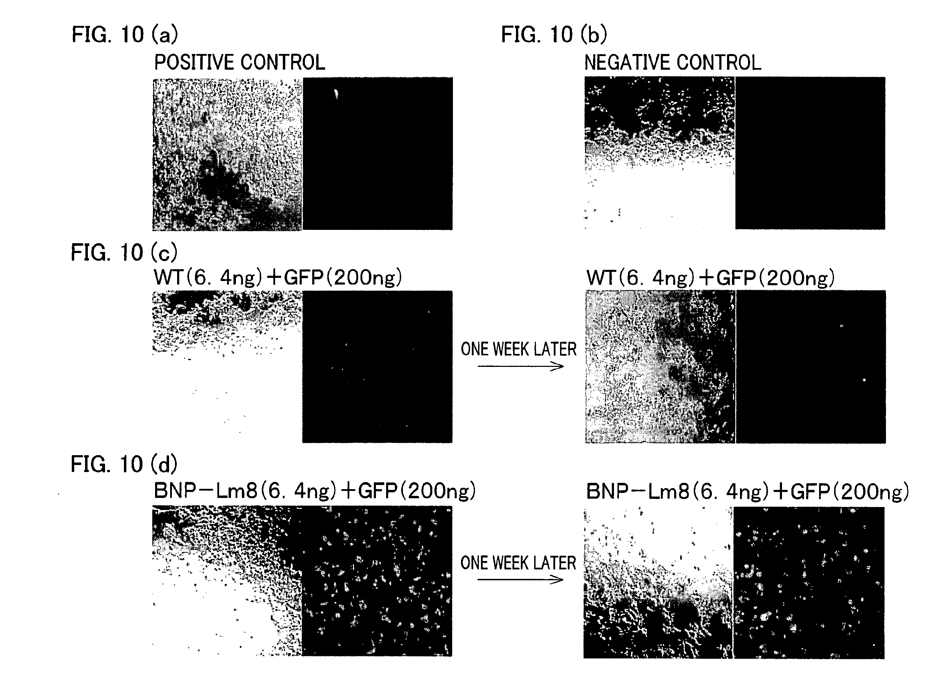

In order to examine preservability of newly collected particles in terms of their ability to transfer genes, particles immediately after the collection were stored for one week at 4°C. After the storage, GFP expression vector DNA was transferred, and the particles were added to a HepG2 cell medium. After 3 days, fluorescence was observed with a confocal microscope. The result is shown in Fig. 10. For each sample, the photograph on the left is a transmission image, and the photograph on the right is a fluorescent image.

In Fig. 10, (a) represents a positive control (containing a compound material of GFP expression vector DNA (200 ng) and FuGene 6 (0.5 µl)), (b) represents a negative control (containing only GFP expression vector DNA (200 ng)), (c) represents a sample containing the wild-type HBsAg particles incorporating the GFP expression vector (wild-type HBsAg (WT) (6.4 ng) + GFP expression vector (200 ng)), immediately after the collection (two photographs on the left) and one week after the collection (two photographs on the right), and (d) represents a sample containing the BNP-Lm8 particles incorporating the GFP expression vector (BNP-Lm8 (6.4 ng) + GFP expression vector (200 ng)), immediately after the collection (two photographs on the left) and one week after the collection (two photographs on the right).

It can be seen that the cells can exhibit green fluorescence of the GFP expression vector even when the GFP expression vector is incorporated in the BNP-Lm8 particles that had been stored for one week at 4°C. That is, the BNP-Lm8 had desirable preservability.

### (8) Hepatocyte-specific gene transfer by the BNP-Lm8

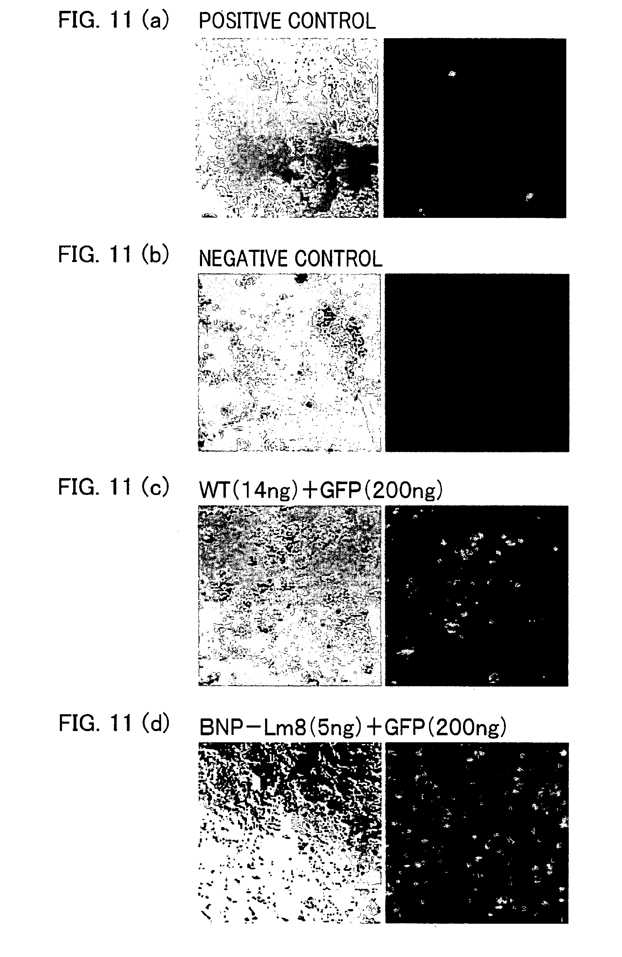

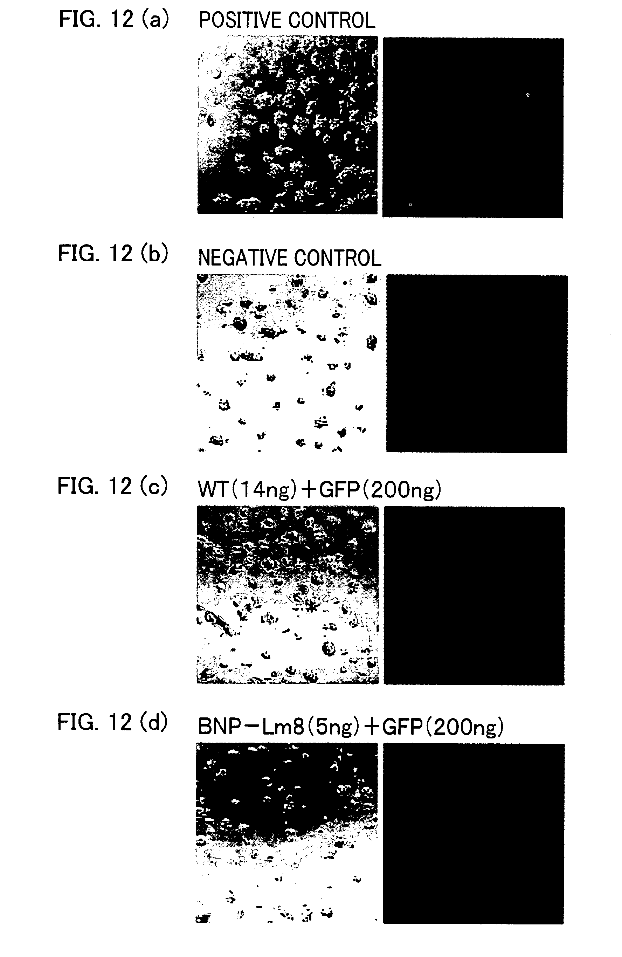

A sample obtained from the HepG2 cells by carrying out an experiment according to the foregoing procedure (Fig. 11) was compared with a sample obtained by using not the HepG2 cells but human colon cancer derived cells WirDr (Fig. 12). In Fig. 11 and Fig. 12, the photographs on the left are phase-contrast images, and the photographs on the right are fluorescent images. Further, in Fig. 11 and Fig. 12, (a) represents a positive control (containing a compound material of GFP expression vector DNA (200 ng) and FuGene 6 (0.5 µl)), (b) represents a negative control (containing only the GFP expression vector DNA), (c) represents wild-type HBsAg particles containing the GFP expression vector (wild-type HBsAg (WT) (14 ng) + GFP expression vector (200 ng)), and (d) represents a sample using the BNP-Lm8 particles containing the GFP expression vector (BNP-Lm8 (5 ng) + GFP expression vector (200 ng)).

It can be seen from this that the HepG2 cells including wild-type HBsAg particles containing the GFP expression vector, and the HepG2 cells including BNP-Lm8 particles containing the GFP expression vector both incorporated the GFP, while the GFP was not incorporated in the human colon cancer derived cells WirDr. The result therefore showed that the human hepatocyte-specific gene transfer was also possible with the BNP-Lm8 particles.

### (9) Gene transfer efficiency using BNP-Lm8 and BNP-Lm7b

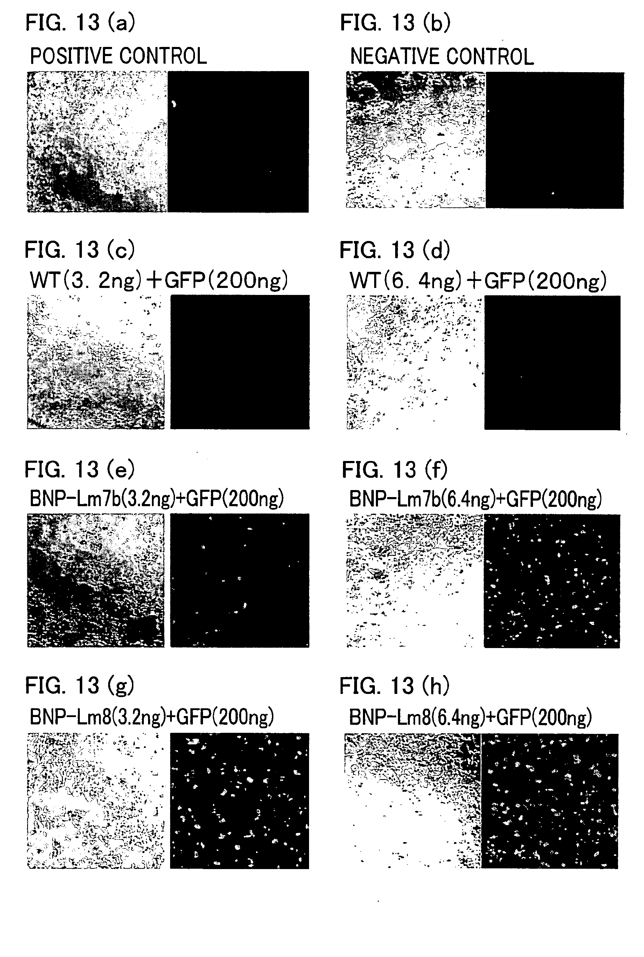

In order to examine transfer efficiency of GFP into cells using GFP-BNP-Lm8 and GFP-BNP-Lm7b, a sample obtained by carrying out the foregoing experiment with the GFP-BNP-Lm8 was compared with a sample obtained by carrying out the foregoing experiment with the GFP-BNP-Lm7b (Fig. 13). In Fig. 13, (a) represents a positive control (containing a compound material of GFP expression vector DNA (200 ng) and FuGene 6 (0.5 µl)), (b) represents a negative control (containing only GFP expression vector DNA), (c) represents a sample including wild-type HBsAg particle containing the GFP expression vector DNA (wild-type HBsAg (WT) (3.2 ng) + GFP expression vector DNA (200 ng)), (d) represents a sample including wild-type HBsAg particles containing the GFP expression vector DNA (wild-type HBsAg (WT) (6.4 ng) + GFP expression vector DNA (200 ng)), (e) represents a sample including BNP-Lm8 particles containing the GFP expression vector DNA (BNP-Lm8 (3.2 ng) + GFP expression vector DNA (200 ng)), (f) represents a sample including BNP-Lm7b particles containing the GFP expression vector DNA (BNP-Lm8 (6.4 ng) + GFP expression vector DNA (200 ng)), (g) represents a sample including BNP-Lm7b particles containing the GFP expression vector DNA (BNP-Lm8 (3.2 ng) + GFP expression vector (200 ng)), and (h) represents a sample including BNP-Lm8 particles containing the GFP expression vector DNA (BNP-Lm8 (6.4 ng) + GFP expression vector (200 ng)).

It can be seen from this that the cell fluorescence, which was strong with the BNP-Lm7b particles containing the GFP expression vector, was even stronger with the BNP-LM8 particles containing the GFP expression vector.

In sum, the HBsAg particles with the substituted Cys residues, particularly the BNP-Lm8, showed stronger fluorescence than the wild-type HBsAg particles, indicating that these particles can sufficiently transfer DNA to cells such as the human hepatocytes and other animal cells, or that these particles can efficiently envelope various substances. Further, the specificity of these particles to the hepatocytes was well preserved. The BNP-Lm7b with the substitution of 7 Cys residues, and the BNP-Lm8 both had stronger fluorescence than the wild-type particles, but the latter showed stronger fluorescence than the former. That is, with the substitution of all of Cys residues considered to be unnecessary in the particle formation, the BNP-Lm8 had higher transfer efficiency of DNA into cells compared with the wild-type particles.

### INDUSTRIAL APPLICABILITY

As described above, the present invention provides hollow nanoparticles that can deliver substances to specific cells. The hollow nanoparticles have a stable particle structure, allowing substances to be transferred into cells with improved efficiency.

A drug prepared according to the invention can be used by a convenient method of intravenous injection to selectively and efficiently deliver disease-treating substances to specific cells or tissues. The invention is a great leap forward from conventional gene therapy in that it does not require any surgical operation, and that the risk of side effect is greatly reduced. The drug is therefore usable in clinical applications in its present form.

## Claims

1. Hollow nanoparticles formed of a particle-forming protein with an ability to recognize a specific cell, wherein the protein contains at least one modified cysteine residue.

2. Hollow nanoparticles as set forth in claim 1, wherein the protein comprises a hepatitis B virus surface-antigen protein.

3. Hollow nanoparticles as set forth in claim 1 or 2, wherein at least one cysteine residue present in a transmembrane region is replaced with a hydrophobic amino acid, and/or at least one cysteine residue present outside or inside the particles is replaced with a hydrophilic amino acid.

4. Hollow nanoparticles as set forth in claim 3, wherein at least one cysteine residue present in a transmembrane region is replaced with an alanine residue, and/or at least one cysteine residue present inside or outside the particles is replaced with a serine residue.

5. Hollow nanoparticles as set forth in any one of claims 2 through 4, wherein the hepatitis B virus surface-antigen protein contains S protein with an amino acid sequence whose cysteine residues 76, 90, 139, 147, 149, 221 and at least one of cysteine residues 137 and 138 of the amino acid sequence from its N-terminus have been replaced.

6. Hollow nanoparticles as set forth in any one of claims 1 through 5, wherein the cysteine residues are modified by mutating a gene that encodes the particle-forming protein and by expressing the mutated gene.

7. Hollow nanoparticles as set forth in claim 6, which are obtained by transforming a eukaryotic cell with a vector including the mutated gene, and by expressing the mutated gene in the eukaryotic cell.

8. Hollow nanoparticles as set forth in claim 7, wherein the eukaryotic cell is an animal cell or a yeast cell.

9. A drug which comprises hollow nanoparticles of any one of claims 1 through 8 in which a substance to be transferred into a cell is encapsulated.

10. A drug as set forth in claim 9, wherein the substance to be transferred into a cell comprises a gene.

11. A therapeutic method using a drug of claim 9 or 10.
